Europäisches Patentamt

European Patent Office

Office européen des brevets

(19)

(11) Publication number: **0 196 116**
**B1**

(12) **EUROPEAN PATENT SPECIFICATION**

(45) Date of publication of patent specification: 24.01.90

(51) Int. Cl.⁵: **A 61 F 2/24**

(21) Application number: **86105918.6**

(22) Date of filing: **29.07.82**

(60) Publication number of the earlier application in accordance with Art. 76 EPC: **0 086 213**

(54) **Frame for a prosthetic heart valve.**

(30) Priority: **17.08.81 US 293667**

(43) Date of publication of application:
**01.10.86 Bulletin 86/40**

(45) Publication of the grant of the patent:
**24.01.90 Bulletin 90/04**

(84) Designated Contracting States:
**AT BE CH DE FR GB LI LU NL SE**

(56) References cited:
**FR-A-2 337 543**
**US-A-3 714 671**
**US-A-4 084 268**
**US-A-4 192 020**

(73) Proprietor: **BAXTER INTERNATIONAL INC. (a Delaware corporation)**
**One Baxter Parkway**
**Deerfield Illinois 60015 (US)**

(72) Inventor: **Lane, Ernest**
**8542 Keel Drive**
**Huntington Beach California 92646 (US)**

(74) Representative: **Day, Jeremy John et al**
**REDDIE & GROSE 16 Theobalds Road**
**London, WC1X 8PL (GB)**

EP 0 196 116 B1

Courier Press, Leamington Spa, England.

## Description

Prosthetic heart valves are used to replace diseased natural heart valves in the aortic, mitral, tricuspid and pulmonary positions in the heart. Examples of such valves are shown in US—A—3714671, US—A—4106129, US—A—4084268 and US—A—4192020. As shown by these patents, a prosthetic heart valve typically includes a frame formed of a wire or a shell valve and leaflets attached to the frame.

US—A—3714671 discloses a prosthetic heart valve having a frame made from three separate wires each being formed along a segment of a circular or elliptical cylindrical surface so that the assembled frame is generally circular or elliptical in shape though not lying in a single plane. Each of the wire arcs has an upstanding portion at each end that blends into a line which is parallel to the axis of the cylinder and has an arcuately dipped intermediate portion. Each wire is in line radially with an end of a succeeding wire and the ends of the wires are secured together by welding.

US—A—4106129 discloses a prosthetic heart valve having a wire frame composed of a single flexible wire preformed to define inverted U-shaped commissure supports merging smoothly with arcuate portions connecting such supports in which the two ends of the wire forming the frame are connected together along one of the arcuate connecting portions by means of a crimped sleeve or coupling.

It is desirable in a prosthetic heart valve having a wire frame that the frame should allow maximum opening of the valve and to this end should have no sharp bends or kinks such as would reduce the valve opening or be incompatible with the tissue with which it will be in contact during use. Moreover, the frame should not have any sharp ends or corners that could damage the material of the valve.

The frame of US—A—3714671 has protruding ends of wires at the points where the three wires are joined together. In the frame of US—A—4106129 joining together of the ends of the wire in a curved portion of the frame introduces difficulties. First, it is very difficult to crimp, without distortion, a sleeve that is bent to conform to the centre and if a straight sleeve is used to join two curved ends of wire, the wire must develop a kink where it enters the sleeve.

Although any means for joining the opposite ends of the wire inherently provide a discontinuity in the frame, this invention provides a frame which the effects of such discontinuity are minimised by providing the join between two straight axially aligned portions of wire.

According to this inention a frame for a prosthetic heart valve comprises:

a wire conformed into an annular configuration and having a plurality of circumferentially shaped apical sections joined by curved sections, each of said apical sections including a reverse bend and first and second straight segments joining the opposite ends of the reverse bend of each apical section to the adjacent curved sections, characterised in that the wire has opposite ends lying in one of said straight segments of the frame and means are provided for joining said opposite ends of said wire together in said one straight segment, with the two ends axially aligned.

To allow attachment to the valve leaflets with a minimum of discontinuities and to reduce the overall axial dimension of the valve without sharp bends in the wire, each of the curved sections is preferably substantially elliptical as viewed in side elevation and forms a portion of an ellipse.

The frame can advantageously be constructed by a method which includes forming a wire into a generally flat pattern, with the wire having a plurality of curved sections joined by apical sections and with each of the apical sections including a reverse bend. The apical sections extend in the same general direction, and the wire has opposite ends. The flat pattern is then formed into an annular configuration, and the opposite ends of the wire are joined to retain the wire in the annular configuration.

To minimise the effect of the discontinuity formed by joining the ends of the wire, the wire terminates in straight sections, which are preferably tangential to the associated reverse bend and the associated curved section, respectively. The joining means can advantageously take the form of a splice.

The frame may be constructed of various biocompatible materials, such as suitable metals, plastics or composite fibrous materials. For example, a suitable cobalt alloy, a polyolefin or a carbon reinforced, non-metallic material may be used.

The wire frame of this invention can advantageously be used in a prosthetic heart valve according to EP—A—0086213.

The invention, together with further features and advantages thereof, may best be understood by reference to the following description taken in connection with the accompanying illustrative drawings, in which

Figure 1 is a perspective view of a bioprosthetic heart valve incorporating a wire frame constructed in accordance with the teachings of this invention.

Figure 2 is a perspective view of a preferred form of wire frame, with the outline of the valve being shown in phantom lines.

Figure 3 is a side elevational view of the wire formed into a flat pattern in the first step of making the wire frame.

Figures 4—6 are line diagrams showing the centerline configuration of the wire frame in front elevation, side elevation and top plan, respectively.

Figure 7 is a perspective view showing the completed wire frame formed into an annular, frusto-conical configuration.

Figure 8 is a fragmentary view partially in section showing one form of splice for attaching the opposite ends of the wire together.

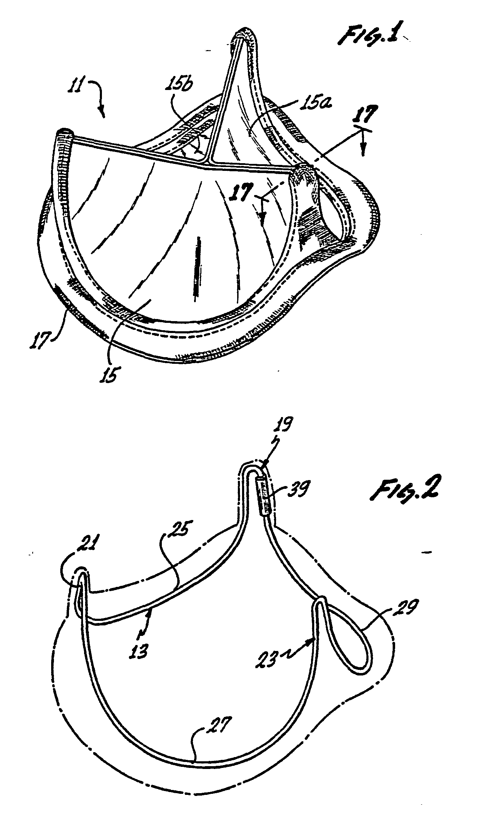

Figure 1 shows a prosthetic heart valve 11

2

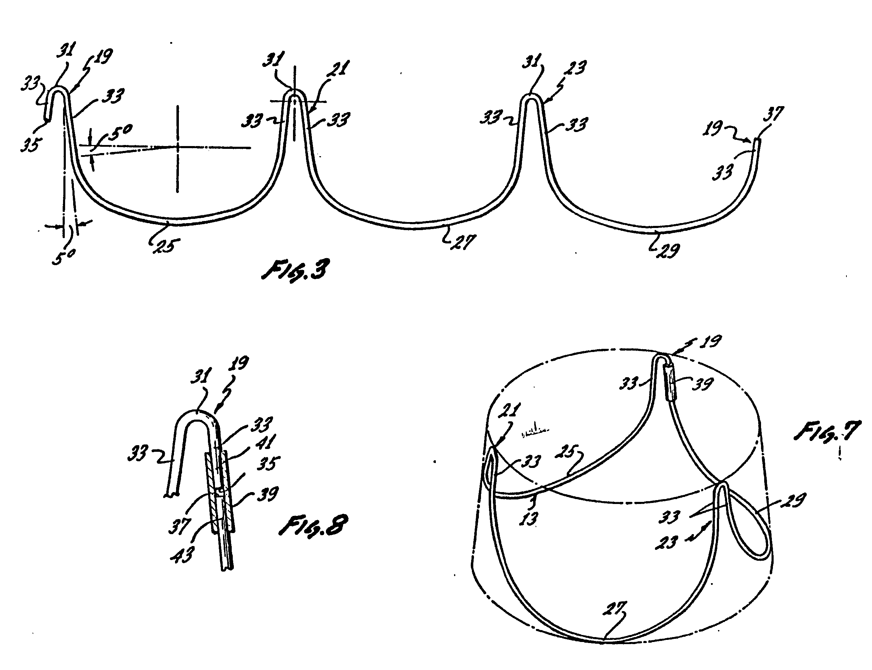

which generally comprises a frame 13 (Figure 2), three identical valve leaflets 15, 15a and 15b, and a suture ring 17. In the embodiment illustrated, the suture ring 17 is scalloped, and accordingly, the heart valve 11 is an aortic heat valve. The valve leaflets 15, 15a and 15b may be of tissue or a synthetic material, such as film polytetrafluoroethylene.

According to a preferred method of making the frame 13, the wire is formed into a flat pattern as shown in Figure 3. The wire has commissure supports 19, 21 and 23 integrally joined by curved sections 25, 27 and 29. Each of the commissure supports is identical and extends in the same direction. Each of the commissure supports 19, 21 and 23 may be considered as forming, or including, an apical section, each of which includes a reverse bend 31 and two tangents 33 joining the opposite ends of the associated reverse bend to the adjacent curved sections. The wire terminates at opposite ends 35 and 37 in straight portions, which together form one of the tangents 33 of the commissure support 19. Each of the tangents 33 is linear, and the tangents 33 of each of the commissure supports diverge as they extend away from the associated reverse bend 31.

Each of the curved sections 25, 27 and 29 is identical and preferably forms a part of an ellipse. For example, in the embodiment illustrated, each of the curved sections 25, 27, 29 extends through 170 degrees, and each of the reverse bends 31 is part circular and also extends through 170 degrees. The included angle formed by an extension of the axes of the tangents 33 of each of the commissure supports thus may be, for example, 10 degrees. This specific dimensional data is given by way of illustration only, and the frame does not require these particular dimensions.

The flat pattern shown in Figure 3 contains no kinks or sharp bends. The tangents 33 blend smoothly into the adjacent reverse bends and curved sections. Similarly, the curved sections 25, 27 and 29 and the reverse bends 31 themselves provide no kinks or sharp corners.

Next, the flat pattern of Figure 3 is formed into an annular, cylindrical configuration, and the opposite ends 35 and 37 are joined together in any suitable manner, such as by splicing, using a cylindrical sleeve 39.

The presence of the straight cylindrical sleeve 39 on the correspondingly straight tangent 33 for splicing the ends 35 and 37 does not alter the configuration of the wire or cause the wire to kink or form a sharp end. By way of contrast, if ends 35 and 37 of the wire terminated in one of the curved sections 25, 27 and 29, or one of the reverse bends 31, the sleeve 39 would have to be carefully correspondingly curved, and crimping of a curved sleeve to avoid kinks would be difficult. If a straight sleeve were used, kinks in the wire would be unavoidable. With this invention, the tangent 33 on which the sleeve 39 is carried, may be considered as including tangent sections 41 and 43 which are spliced together by the sleeve 39.

According to the preferred method, the final step in constructing the frame 13 is to form the cylindrical annular configuration into a frusto-conical annular configuration as shown in Figures 2 and 7. In this configuration, the commissure supports 19, 21 and 23 are inclined toward each other as they extend away from their associated curved sections 25, 27 and 29. By way of example, the configuration of the frame 13 in Figure 2 is a frustum of a right circular cone with each of the commissure supports 19 being inclined radially inwardly at about 5 degrees. Although the wire is deformed from the flat configuration of Figure 3 of the frusto-conical configuration of Figure 2 (or Figure 7), the curved sections 25, 27 and 29 remain in an elliptical configuration as viewed in side elevation, the tangents 33 remain straight and the reverse bends 31 remain part circular. However, the aspect ratio of the partial ellipses formed by the curved sections 25, 27 and 29 is altered somewhat. The frame 13, including the commissure supports 19, 21 and 23, is resiliently flexible and can better accommodate shock loading.

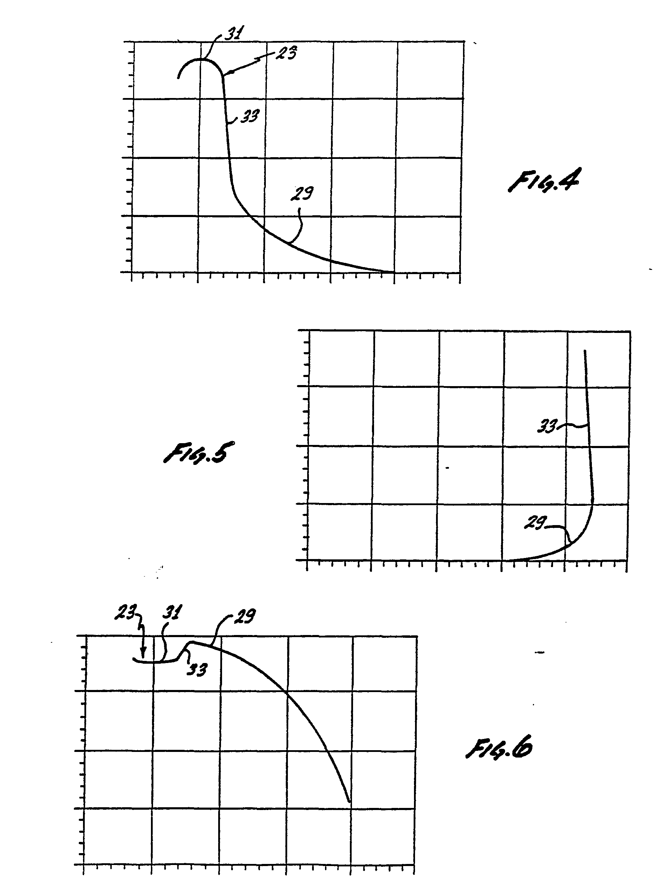

Figures 4—6 illustrate how the wire frame 13 provides a smoothly curved contour at all locations along the frame, and in particular, along and adjacent the commissure support 23, it being understood that the commissure supports 19 and 21 are identical to the commissure support 23. Figures 4—6 show the center line of the wire in line diagram form, and it can be seen that smooth curves exist in all three views. Figure 4, which is a side elevational view, illustrates one half of the part elliptical configuration of the curved section 29.

**Claims**

1. A frame (13) for a prosthetic heart valve (11) comprising:
a wire formed into an annular configuration and having a plurality of circumferentially spaced apical sections (19, 21, 23) joined by curved sections (25, 27, 29), each of said apical sections (19, 21, 23) including a reverse bend (31) and first and second straight segments (33) joining the opposite ends of the reverse bend (31) of such apical section (19, 21, 23) to the adjacent curved section (25, 27, 29), respectively: and characterised by said wire having opposite ends (35, 37) lying on one of said straight segments (33) and means (39) joining said opposite end (35, 37) of said wire together in said one straight segment (33) with the two ends axially aligned.

2. A frame according to Claim 1, wherein each of said curved sections (25, 27, 29) forms a portion of an ellipse as viewed in side elevation.

3. A frame according to Claim 2, wherein each of the curved sections (25, 27, 29) extends for 170 degrees.

4. A frame according to any one of Claims 1 to 3, wherein an extension of the axes of the segments (33) joined to one of the reverse bends (31) forms an included angle of 10 degrees.

5. A frame according to any one of Claims 1 to 4, wherein said straight segments (33) are tangential to both the reverse bends (31) and the curved sections (25, 27, 29).

6. A frame according to any one of Claims 1 to 5, wherein said frame is of a frusto-conical annular configuration, said apical section (19, 21, 23) being inclined radially inwardly of the frame (11).

7. A frame according to any one of Claims 1 to 6, wherein the joining means (39) is a cylindrical sleeve.

8. A method for making a frame according to Claim 1, which comprises forming a wire into a flat pattern comprising a plurality of reverse bends and a plurality of curved sections connected to the reverse bends by straight sections, the opposed ends of the wire being straight portions, forming the flat pattern into an annular cylindrical configuration to bring the two opposed ends of the wire together in axial alignment and joining the two ends of the wire.

**Patentansprüche**

1. Rahmen (13) für ein Kunstherzventil (11) mit einem Draht, dem eine Ringform gegeben ist und der eine Vielzahl von in Umfangsrichtung beabstandeten spitzen Abschnitten (19, 21, 23) aufweist, die durch gekrümmte Abschnitte (25, 27, 29) miteinander verbunden sind, wobei jeder Spitzenabschnitt (19, 21, 23) eine Rückbiegung (31) und ein erstes und ein zweites gerades Segment (33) aufweist, die die gegenüberliegenden Enden der Rückbiegung (31) der Spitzenabschnitte (19, 21, 23) mit dem benachbarten gekrümmten Abschnitt (25, 27, 29) jeweils verbinden, dadurch gekennzeichnet, daß der Draht gegenüberliegende Enden (35, 37) aufweist, die auf einem der geraden Segmente (33) liegen, und Einrichtungen (39) die gegenüberliegenden Enden (35, 37) des Drahtes in dem einen geraden Segment (33) so miteinander verbinden, daß die beiden Enden axial in einer Linie zueinander ausgerichtet sind.

2. Rahmen nach Anspruch 1, bei dem jeder gekrümmte Abschnitt (25, 27, 29) gesehen in einer Seitenansicht eine Ellipse bildet.

3. Rahmen nach Anspruch 2, bei dem jeder gekrümmte Abschnitt (25, 27, 29) über 170° verläuft.

4. Rahmen nach einem der Ansprüche 1 bis 3, bei dem die Verlängerungen der Achsen der Segmente (33), die mit einer der Rückbeigungen (31) verbunden sind, einen Winkel von 10° einschließen.

5. Rahmen nach einem der Ansprüche 1 bis 4, bei dem die geraden Segmente (33) sowohl zu den Rückbiegungen (31) als auch zu den gekrümmten Abschnitten (25, 27, 29) tangential verlaufen.

6. Rahmen nach einem der Ansprüche 1 bis 5, bei dem der Rahmen eine kegelstumpfförmige Ringform hat, wobei die Spitzenabschnitte (19, 21, 23) vom Rahmen (11) radial schräg nach innen verlaufen.

7. Rahmen nach einem der Ansprüche 1 bis 6, bei dem die Verbindungseinrichtung (29) eine zylidrische Hülse ist.

8. Verfahren zum Herstellen eines Rahmens nach Anspruch 1, bei dem einem Draht eine ebene Form mit einer Vielzahl von Rückbiegungen und einer Vielzahl von gekrümmten Abschnitten gegeben wird, die über gerade Abschnitte mit den Rückbiegungen verbunden sind, wobei die gegenüberliegenden Enden des Drahtes gerade Teile sind, die ebene Form in eine zylindrische Ringform gebracht wird, um die beiden gegenüberliegenden Enden des Drahtes in eine axiale Ausrichtung zueinander zu bringen, und die beiden Enden des Drahtes verbunden werden.

**Revendications**

1. Support pour une prothèse de valvule cardiaque (11) comprenant:

un fil formé en une configuration annulaire et ayant un certain nombre de sections apicales circonférentiellement espacées (19, 21, 23) jointes par des sections courbées (25, 27, 29), chacune desdites sections apicales (19, 21, 23) ayant un coude inverse (31) et des premier et second segments droits (33) joignant les extrémités opposées du coude inverse (31) de cette section apicale (19, 21, 23) à la section courbée adjacente (25, 27, 29), respectivement, et caractérisé en ce que ledit fil a des extrémités opposées (35, 37) qui se trouvent sur l'un desdits segments droits (33) et des moyens (39) joignant lesdites extrémités opposées (35, 37) dudit fil dans ledit segment droit (33) avec les deux extrémités axialement alignées.

2. Support selon la revendication 1, où chacune desdites sections courbées (25, 27, 29) forme une portion d'une ellipse en regardant en élévation latérale.

3. Support selon la revendication 2, où chacune des sections courbées (25, 27, 29) s'étend sur 170 degrés.

4. Support selon l'une quelconque des revendications 1 à 3 où une extension des axes des segments (33) joints à l'un des coudes inverses (31) forme un angle inclus de 10 degrés.

5. Support selon l'une quelconque des revendications 1 à 4, où ledit segment droit (33) sont tangentiels aux coudes inverses (31) et à la section courbée (25, 27, 29).

6. Support selon l'une quelconque des revendications 1 à 5, où ledit support est de configuration annulaire tronconique, ladite section apicale (19, 21, 23) étant inclinée radialement vers l'intérieur du support (11).

7. Support selon l'une quelconque des revendications 1 à 6, où le moyen de jonction (39) est un manchon cylindrique.

8. Méthode de fabrication d'un support selon la revendication 1, qui consiste à former un fil en un schéma plat comprenant un certain nombre de coudes inverses et un certain nombre de sections courbées reliées aux coudes inverses par des sections droites, les extrémités opposées du fil étant des portions droites, à former le schéma plat en une configuration cylindrique annulaire pour rassembler les deux extrémités opposées du fil en alignement axial et à joindre les deux extrémités du fil.

Fig.1

Fig.2

Fig.3

Fig.8

Fig.7

EP 0 196 116 B1

_FIG.4_

_FIG.5_

_FIG.6_